# EUROPEAN PATENT SPECIFICATION

(11) **EP 0 552 271 B1**
(45) Date of publication and mention of the grant of the patent: **24.04.1996**
(21) Application number: 91919353.2
(22) Date of filing: 08.10.1991
(51) Int. Cl.: A61F 13/06, A61F 13/02

(54) **WOUND DRESSING**
WUNDVERBAND
PANSEMENT POUR PLAIES

(30) Priority: 09.10.1990 GB 9021910
(43) Date of publication of application: 28.07.1993
(73) Proprietor: Smith & Nephew plc, London WC2R 3BP (GB)
(72) Inventor: PENROSE, Jane Edith, 5 Chester Avenue,, Kingston-upon-Hull HU5 2HJ (GB)
(74) Representative: Gilholm, Stephen Philip
(86) International application number: GB9101745
(87) International publication number: WO9205756

(56) References cited:
- EP-A- 0 174 803
- WO-A-91/01706
- US-A- 3 322 118
- US-A- 3 670 725
- US-A- 3 693 619

## Description

The present invention relates to dressings and in particular dressing for covering limb extremities, and dressing material blanks from which the dressings can be formed.

Conventional dressings for the treatment of wounds or pressure sores are essentially flat dressings which are only sufficiently absorbent to absorb wound exudate and conformable to be applied to extensive flat or curved areas of the body. These flat dressings, however, are not very suitable for use on protruding areas of the body such as elbows, heels and the tops of fingers or toes.

United States Patent No. 3937218 discloses protective cushion pads for prevention of pressure sores on patients who are confined to bed for extended periods. However, there is no teaching in this patent that the pads can be used in the treatment of wounds as a wound or pressure sore dressing or that the pads are aborbent or that such pads are extensible to enable them to be used as conformable absorbent wound dressings.

European Patent No.0174803 describes adhesive wound dressings which comprise a moisture vapour permeable film intermediate layer coated on its wound facing surface with an adhesive layer and provided on its non-wound facing surface with an outer foam layer. Such dressings are however substantially flat and are not suitable for use on protruding areas of the body such as elbows, heels and the tops of fingers or toes.

Conformable absorbent dressings have now been found which have a shape which is suitable for treating wounds or pressure sores on protruding areas or parts of the body and which can easily be formed from a flat dressing blank.

In accordance with the present invention there is provided a conformable wound dressing comprising a body facing layer, an outer layer and an extensible intermediate layer characterised in that the body facing layer is an apertured wound contacting elastomeric material, the outer layer is a bacteria impermeable moisture vapour transmitting elastomeric film and the intermediate layer is an absorbent material; the apertured wound contacting elastomeric material has a concave wound contacting surface and the outer film has an outwardly facing convex surface.

The term "conformable" as used herein with respect to a dressing of the invention means a dressing which is capable following the contours of the body and also the movement of the skin at areas to which it has been applied.

The concavo-convex shape or cup-like shape of the dressing of the invention renders it highly suitable for use on protruding parts of the body such as an elbow, heel, toe or finger (includes thumb).

These protruding areas or parts of the body are prone to injury by knocks, abrasion, pressure or a combination of two of these conditions. Such injury may cause a wound or a pressure sore on the protruding area.

The conformable and absorbent nature of the dressings of the invention renders them highly suitable for use on such wounds and pressure sores.

One favoured dressing of the invention is therefore a wound dressing.

Another favoured dressing of the invention is therefore a pressure sore dressing. The size and shape of the dressing can be adapted to the protruded area to which the dressing is to be applied.

Preferred dressings of the invention are adapted to be applied to the heel or elbow.

The absorbent material used in dressings of the invention will be extensible, aptly soft and flexible and preferably also resilient to provide the dressing with a cushioning property.

Suitable absorbent materials of this type include absorbent polymer foam layers or sheets and absorbent non-woven fabrics containing bonded high loft fibres and absorbent fibres (if not high loft fibres) such as cellulosic fibres for example cotton or viscose rayon fibres.

Favoured absorbent materials for use in the invention are absorbent foam layers such as open celled foam layers of an elastomeric polymer such as polyurethane. Preferred absorbent materials, however, are absorbent foam layers of a hydrophilic polymer such as hydrophilic polyurethane.

Hydrophilic polymer foam layers can provide the dressing with a high absorbent capacity.

Suitable hydrophilic polyurethane foams for use in the invention are disclosed in European Patent Application No. 0299122 and European Patent No. 0059049.

The dressing of the invention can advantageously comprise a non-adherent wound facing layer. Suitable non-adherent layers include apertured elastomeric layers such as flexible apertured films and flexible nets having a non fibrous surface. Favoured layers of this type are nets or apertured films or elastomers. Preferred wound facing layers however are nets of an elastomer such as thermoplastic polyurethane and A-B-A type block copolymers where A is polystyrene and B is polybutadiene or polyisoprene.

A wound facing net of elastomer can render the dressing of the invention highly conformable to wound or sore surfaces. Suitable non-adherent wound facing layers for use in the invention are disclosed in the hereinbefore mentioned European Patent No. 0059049.

The outer layer of the dressing of the invention can comprise a flexible continuous film to provide a barrier to the penetration of aqueous liquids and bacteria, ie. a bacteria and aqueous liquid impermeable layer.

The barrier film is also moisture vapour permeable to allow evaporation of aqueous fluid under the dressing.

Favoured barrier films of this type for use in the invention include flexible films of polyurethane, such as thermoplastic polyether-polyurethane, blends thereof with an incompatible polymer such as polystyrene, polyether-ester and polyether ester block copolymers and hydrophilic polyurethane films.

Suitable barrier films for use in the invention are disclosed in the hereinbefore mentioned European Patent No. 0059049. Suitable hydrophilic polyurethane films for use in the invention are disclosed in European Patent No. 0123465.

Favoured absorbent materials for use in the invention comprise a laminate of a non-adherent wound facing layer, an intermediate absorbent layer and an outer continuous film barrier layer which is a bacteria and aqueous liquid impermeable layer.

In preferred absorbent materials for use in the invention the non-adherent wound facing layer comprises a net or apertured film of elastomer, the absorbent layer comprises a flexible hydrophilic polymer such as hydrophilic polyurethane foam and the outer continuous film barrier layer is moisture vapour permeable layer.

Suitable favoured and preferred absorbent materials of this type and methods for their production are disclosed in the hereinabove mentioned European Patent No. 0059049.

A dressing comprising the favoured and preferred laminate absorbent materials hereinbefore mentioned has a number of advantages over a conventional dressing. The conformable nature of the dressing allows it to maintain close contact to a body surface to which it is applied thereby inhibiting any pooling of non-absorbed fluid from an exuding wound or pressure sore. The absorbent capacity of the dressing absorbs large amounts of exudate from an exuding wound or sore. The barrier properties of the dressing inhibits penetration of pathogenic bacteria or aqueous liquid through the dressing to the wound or sore site and can also inhibit egress of exudate from the wound or sore site to the outer surface of the dressing.

The resilient nature of the dressing allows the dressing to cushion a wound or sore area against pressure and also allows the dressing to quickly recover its initial thickness after being compressed.

The thickness of a dressing of the invention can suitably be at least 0.5mm and can preferably be at least 1mm.

The thickness of the dressing can suitably also be not greater than 20mm and can preferably be not greater than 15mm. Typically a thickness of 1mm to 12mm has been found satisfactory.

The moisture vapour permeability properties of the dressing can allow absorbed exudate to evaporate thereby increasing absorbent capacity of the dressing. Such permeability properties will also allow intact skin under the dressing to breathe thereby inhibiting maceration thereof by the build up of moisture in the skin.

The moisture vapour transmission rate of the dressing should be sufficiently high to allow these advantages to occur but sufficiently low to prevent the wound drying out. The moisture vapour transmission rate (MVTR) of the dressing can suitably be greater than 300gm⁻² and can preferably be greater than 500gm⁻² 24h⁻¹ at 100 to 20% relative humidity difference and at 37°c. The moisture vapour transmission rate values hereinafter are abbreviated to gm⁻².

Depending on the use required for the dressing the MVTR of the dressing can suitably be less than 3000gm⁻² and can preferably be less than 2500gm⁻². Alternatively the MVTR can be at least 2500gm⁻² and may be less than 14000gm⁻².

A dressing with these MVTR properties can allow moist healing of a wound or sore and when suitably high can provide a soothing cooling environment which relieves pain.

Moisture vapour transmission rate (MVTR) of the dressing material can be determined by the payne cup method. In this method discs of the dressing material that is a laminate comprising for example a net, foam and outer barrier layer, to be tested are clamped over Payne permeability cups (flanged metals cups) using sealing rings and screw clamps. The exposed surface of the test sample is 10cm². Each cup contains approximately 10ml of distilled water.

After weighing the cups are placed in a fan assisted electric oven maintained at 37°C. The relative humidity difference within the oven is maintained at approximately 10% by placing 1kg of anhydrous 3-8 mesh calcium chloride on the floor of the oven.

The cups are removed after 24 hours, allowed to cool for 20 minutes and reweighed. The MVTR of the test material is calculated from the weight loss and expressed in units of grams of weight per square metre per 24 hours, at 37°c at 100-10% relative humidity difference.

The combination of the MVTR and non-adherent properties of the dressing allow the dressing to be left on the wound or sore site until healed and then removed without disrupting the healed wound or sore surface.

In another aspect the present invention provides a dressing material in the form of a blank having lobate configuration.

According an embodiment of the invention, the dressing material comprise a single blank of the material having two lobes joined together by a common neck region. The concavo-convex shape is formed by folding the blank at the neck region and adhering the edges of each lobe which are adjacent the folded neck region to each other. In adhering these edges together the blank is maintained in the form of a concavo-convex shape.

Alternatively, the dressing material may be separate pieces or blanks of the material each of a lobate configuration. On joining the two pieces together at a part of the edge region the composite structure deforms to assume a concavo-convex shape.

The edges of the material can be joined by any suitable convenient method. Suitable methods include adhesive bonding, heat sealing and fixing by adhesive tape.

A preferred embodiment of joining the edges comprises a heat sealing method such as an impulse induction or radio frequency heat sealing method.

During the joining it is preferred that the edges of the sides are abutted together to provide a substantially smooth or even join.

The concavo-convex shape of a dressing of the invention comprising a laminate can conveniently be formed by abutting a first edge of the laminate with a second edge of the laminate and sealing the edges together along a curved line.

Each of the edges will normally be an edge of a blank formed by a single sheet of the laminate.

The invention will now be illustrated by reference to the following drawings in which:

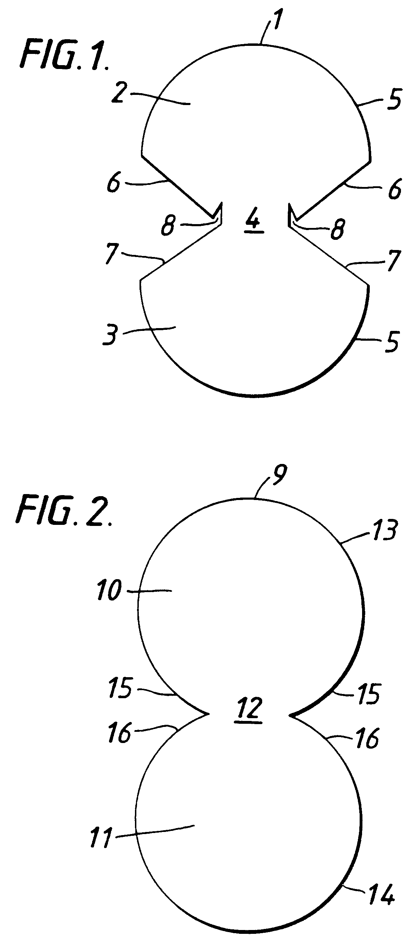

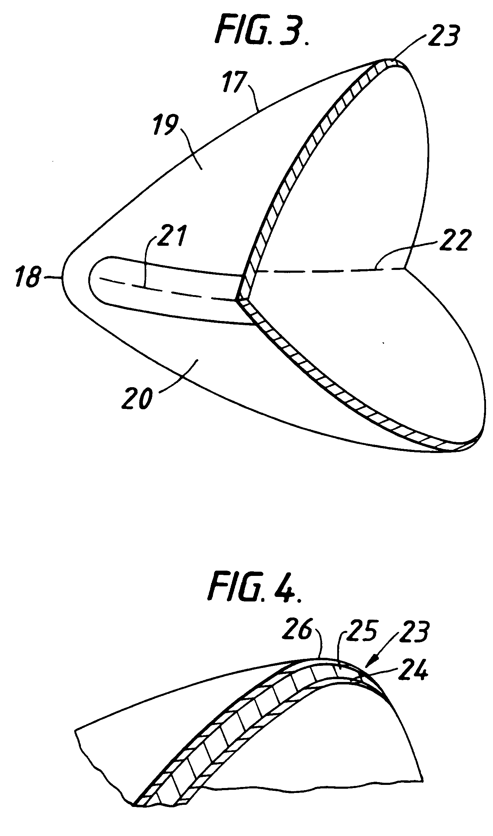

Figure 1 is a plan view of a blank of the invention, Figure 2 is a plan view of a different blank of the invention, Figure 3 is a perspective view of a dressing of the invention formed from the blank shown in Figure 1, Figure 4 is an enlarged version of an edge portion of Figure 3.

Figure 1 shows a blank 1 of the invention made of a soft, flexible, absorbent material with the wound facing surface uppermost. Blank 1 has two portions 2, 3 connected by neck portion 4.

In the embodiment shown in Figure 1 portions 2, 3 have a similar bulbous shape and size. Portions 2, 3 each have rounded ends 5 and a pair of straight sides 6, 7 adjacent to neck portion 4.

Blank 1 can be folded (inwardly of the wound facing surface) about neck portion 4 and opposed sides 6, 7 of portions 2, 3 joined together to form a concavo-convex shaped dressing. Portion 2 of Blank 1 has re-entrant cut out portions 8 either side of neck portion to compensate for the thickness of the absorbent material.

Figure 2 shows a blank 9 similar to that shown in Figure 1 having two portions 10 and 11 connected by a neck portion 12. Portions 10 and 11 have a similar circular shape and size. Both these portions have rounded ends 13 and 14 and a pair of sides 15 and 16 adjacent to the neck portion. However, sides 15 and 16 are curved sides instead of straight sides in the blank of Figure 1.

In other embodiments of the invention the blank portions 2, 3 can be adapted in size and shape to the type of dressing which it will form, for example, in a blank for forming a heel dressing portion 2 which is adapted to cover the back and side of the heel could be wider than that of portion 3 which is adapted to cover the bottom of the heel.

Figure 3 shows a dressing of the invention formed from the blank of Figure 1. Dressing 17 has a flattened concavo-convex or cup shape. Dressing 17 has folded peripheral region 18 and two outwardly extending skirts 19, 20 which are joined at two side regions 21, 22. The angle subtended by the skirts shown in Figure 2 is less 90°. Such an angle can be adapted to the type of dressing eg. an elbow dressing is likely to have a much higher peripheral angle than a finger dressing.

Figure 4 is an enlarged version of the edge of the skirt 19 of dressing 17 showing the absorbent material 23 of dressing 17. Absorbent material 23 has wound facing layer 24, absorbent layer 25 and an outer layer 26.

In a further aspect the present invention provides a method of treating a wound on a protruding part of the body of a patient which comprises applying a dressing over the protruding part to contact the surface of the wound thereon wherein the dressing comprises a conformable wound dressing comprising a body facing layer, an outer layer and an extensible intermediate layer characterised in that the body facing layer is an apertured wound contacting elastomeric material, the outer layer is a bacteria impermeable moisture vapour transmitting elastomeric film and the intermediate layer is an absorbent material and; apertured wound contacting elastomeric material has a concave wound contacting surface and the outer film has an outwardly facing convex surface.

In yet a further aspect the present invention provides a method of treating a pressure sore on a protruding part of the body of a patient which comprises applying a dressing over the protruding part to contact the surface of the pressure sore thereon wherein the dressing comprises a conformable wound dressing comprising a body facing layer, an outer layer and an extensible intermediate layer characterised in that the body facing layer is an apertured wound contacting elastomeric material, the outer layer is a bacteria impermeable moisture vapour transmitting elastomeric film and the intermediate layer is an absorbent material; the apertured wound contacting elastomeric material has a concave wound contacting surface and the outer film has an outwardly facing convex surface.

When applying the dressing the wound contacting surface of the dressing makes contact with the surface of the wound or sore on the protruded area.

The dressing can be maintained on the protruded area by any suitable means. Such means include a bandage such as tubular bandage, an adhesive tape or an adhesive coating on the wound facing surface of the dressing.

Such an adhesive coating can extend over a part or whole of the wound facing surface of the dressing, in a favoured dressing of the invention the adhesive coating is located on the wound facing surface thereof on the peripheral area of the lobate regions of the dressings. Such dressings advantageously have a wound. contacting area free when the dressing comprises an apertured layer such as a net or apertured film. The adhesive coating can advantageously be on the unapertured area thereof to render the adhesive layer moisture vapour permeable.

The adhesive used in the invention will normally be a pressure sensitive adhesive which is suitable for contact with skin.

Suitable adhesive include polyvinyl alkylether, acrylate ester copolymer and polyurethane gel adhesives. Suitable polyvinyl alkylether in European Patent Nos. 35399 and 51935 and United Kingdom Patent No. 1280631. Apt adhesives of this type are polyvinyl ethyl ether adhesives and copolymers of 2 ethyl hexyl acrylate, butyl acrylate and acrylic acid.

Suitable polyurethane gel adhesives are disclosed in European Patent 123465. Such gel adhesives advantageously have a low adhesion to wound and skin surfaces and therefore can be easily removed therefore without causing damage to the skin or the wound.

## Claims

1. A conformable wound dressing (17) comprising a body facing layer (24), an outer layer (26) and an extensible intermediate layer (23) characterised in that the body facing layer (24) is an apertured wound contacting elastomeric material, the outer layer (26) is a bacteria impermeable moisture vapour transmitting elastomeric film and the intermediate layer (23) is an absorbent material; the apertured wound contacting elastomeric material has a concave wound contacting surface and the outer film has an outwardly facing convex surface.

2. A dressing as claimed in claim 1 in which the body facing layer, intermediate absorbent material layer and outer layer are a laminate.

3. A dressing as claimed in claims 1 or 2 in which the absorbent material comprises a sheet of polymer foam.

4. A dressing as claimed in any one of claims 1 to 3 in which the body facing layer comprises a polyurethane.

5. A dressing as claimed in any one of claims 1 to 4 in which the body facing layer comprises a net of elastomer.

6. A dressing as claimed in any one of claims 1 to 4 wherein the absorbent material comprises a hydrophilic polymer foam.

7. A dressing as claimed in claim 5 wherein the elastomer is a polyurethane.

8. A dressing as claimed in any one of claims 1 to 6 wherein the outer layer comprises a moisture vapour transmitting polyurethane film.

9. A dressing as claimed in claim 8 wherein the polyurethane film has a moisture transmission rate of at least 300 gm⁻² at 100 to 10% relative humidity difference at 37°C.

10. A dressing as claimed in any of claims 1 to 9 which comprises an adhesive layer on its wound facing surface.

11. A dressing as claimed in any of claims 1 to 10 which has a moisture vapour transmission rate of at least 300 gm⁻² at 100 to 10% relative humidity difference at 37°C.

12. A dressing as claimed in any of claims 1 to 9 which is a wound or pressure sore dressing.

13. A dressing as claimed in any of claims 1 to 10 which is adapted to be applied to the heel.

14. A dressing as claimed in any one of claims 2 to 13 wherein the concavo-convex shape is formed by abutting a first edge of the laminate with a second edge of the laminate and sealing the edges together along a curved line.

15. A dressing as claimed in claim 14 wherein each of said edges is an edge on a blank formed by a single sheet of said laminate.

16. A dressing material in the form of a blank having a lobate configuration.

17. A dressing material as claimed in claim 13 in which the blank has two lobes joined together by a common neck portion.

18. A dressing as claimed in any one of claims 2 to 17 wherein each of said first and second edges are, respectively, an edge of separate sheets of a laminate.

19. A dressing as claimed in any one of claims 14 to 18 wherein the edges are sealed by heat sealing.

## Patentansprüche

1. Anpaßbarer Wundverband (17), welcher eine dem Körper zugekehrte Lage (24), eine äußere Lage (26) und eine dehnbare Zwischenlage (23) umfaßt, dadurch gekennzeichnet, daß die dem Körper zugekehrte Lage (24) ein mit Öffnungen versehenes, die Wunde berührendes Elastomermaterial, die äußere Lage (26) eine bakterienundurchlässige, wasserdampfdurchlässige Elastomerfolie und die Zwischenlage (23) ein absorbierendes Material ist und das mit Öffnungen versehene, die Wunde beruhrende Elastomermaterial eine konkave, die Wunde berührende Oberfläche und die äußere Folie eine nach außen gerichtete konvexe Oberfläche aufweist.

2. Verband nach Anspruch 1, bei welchem die dem Körper zugekehrte Lage, die Zwischenlage aus absorbierendem Material und die äußere Lage ein Laminat bilden.

3. Verband nach Anspruch 1 oder 2, bei welchem das absorbierende Material eine Schicht aus Polymerschaumstoff umfaßt.

4. Verband nach einem der Ansprüche 1 bis 3, bei welchem die dem Körper zugekehrte Lage ein Polyurethan umfaßt.

5. Verband nach einem der Ansprüche 1 bis 4, bei welchem die dem Körper zugekehrte Lage ein Netz aus Elastomer umfaßt.

6. Verband nach einem der Ansprüche 1 bis 4, bei welchem das absorbierende Material einen hydrophilen Polymerschaumstoff umfaßt.

7. Verband nach Anspruch 5, bei welchem das Elastomer ein Polyurethan ist.

8. Verband nach einem der Ansprüche 1 bis 6, bei welchem die äußere Lage eine wasserdampfdurchlässige Polyurethanfolie umfaßt.

9. Verband nach Anspruche 8, bei welchem die Polyurethanfolie eine Wasserdampfdurchlässigkeit von wenigstens 300 gm⁻² bei 100 bis 10% relativem Feuchtigkeitsunterschied bei 37°C hat.

10. Verband nach einem der Ansprüche 1 bis 9, der eine Klebstoffschicht auf seiner der Wunde zugekehrten Oberfläche aufweist.

11. Verband nach einem der Ansprüche 1 - 10, der eine Wassserdampfdurchlässigkeit von wenigstesn 300 gm⁻² bei 100 bis 10% relativem Feuchtigkeitsunterschied bei 37° C hat.

12. Verband nach einem der Ansprüche 1 - 9, der ein Wund- oder Dekubitusverband ist.

13. Verband nach einem der Ansprüche 1 bis 10, der an die Verwendung auf der Ferse angepaßt ist.

14. Verband nach einem der Ansprüche 2 bis 13, bei welchem die die konkav-konvexe Form dadurch gebildet wird, daß man eine erste Kante des Laminats und eine zweite Kante des Laminats aneinander anstoßen läßt und die Kanten entlang einer gekrümmten Linie verschweißt.

15. Verband nach Anspruch 4, bei welchem jede der Kanten eine Kante an einem Vorformling ist, die durch eine einzelne Schicht des Laminats gebildet wird.

16. Verbandmaterial in Form eines Vorformlings mit der Konfiguration eines Lappens.

17. Verbandmaterial nach Anspruch 13, bei welchem der Vorformling zwei durch einen gemeinsamen eingezogenen Bereich miteinander verbundene Lappen aufweist.

18. Verband nach einem der Ansprüche 2 bis 17, bei welchem jede der beiden Kanten eine Kante einer separaten Schicht eines Laminats ist.

19. Verband nach einem der Ansprüche 14 bis 18, bei welchem die Kanten miteinander verschweißt sind.

## Revendications

1. Pansement pour plaies épousant les formes (17) comprenant une couche (24) faisant face au corps, une couche extérieure (26) et une couche intermédiaire extensible (23), caractérisé en ce que la couche (24) faisant face au corps est un matériau élastomère percé d'ouvertures en contact avec la plaie, la couche extérieure (26) est un film élastomère transmettant la vapeur d'eau imperméable aux bactéries et la couche intermédiaire (23) est un matériau absorbant; le matériau élastomère percé d'ouvertures en contact avec la plaie présente une surface concave en contact avec la plaie et le film extérieur présente une surface convexe dirigée vers l'exterieur.

2. Pansement suivant la revendication 1, dans lequel la couche faisant face au corps, la couche intermédiaire de matériau absorbant et la couche extérieure sont un feuilleté.

3. Pansement suivant les revendications 1 ou 2, dans lequel le matériau absorbant comprend une feuille de mousse de polymère.

4. Pansement suivant l'une quelconque des revendications 1 à 3, dans lequel la couche faisant face au corps comprend un polyuréthane.

5. Pansement suivant l'une quelconque des revendications 1 à 4, dans lequel la couche faisant face au corps comprend un filet d'élastomère.

6. Pansement suivant l'une quelconque des revendications 1 à 4, dans lequel le matériau absorbant comprend une mousse de polymère hydrophile.

7. Pansement suivant la revendication 5, dans lequel l'élastomère est un polyuréthane.

8. Pansement suivant l'une quelconque des revendications 1 à 6, dans lequel la couche extérieure comprend un film de polyuréthane transmettant la vapeur d'eau.

9. Pansement suivant la revendication 8, dans lequel le film de polyuréthane a un taux de transmission de vapeur d'eau d'au moins 300 g/m² pour une différence d'humidité relative de 100 à 10% à 37°C.

10. Pansement suivant l'une quelconque des revendications 1 à 9, qui comprend une couche adhésive sur sa surface faisant face à la plaie.

11. Pansement suivant l'une quelconque des revendications 1 à 10, qui a un taux de transmission de vapeur d'eau d'au moins 300 g/m² pour une différence d'humidité relative de 100 à 10% à 37°C.

12. Pansement suivant l'une quelconque des revendications 1 à 9, qui est un pansement pour plaies ou pour escarres.

13. Pansement suivant l'une quelconque des revendications 1 à 10, qui est adapté pour être appliqué sur le talon.

14. Pansement suivant l'une quelconque des revendications 2 à 13, dans lequel la forme concavo-convexe est formée par mise en contact bord-à-bord d'un premier bord du feuilleté avec un second bord du feuilleté et soudure des bords le long d'une ligne incurvée.

15. Pansement suivant la revendication 14, dans lequel chacun de ces bords est un bord d'une galette formée par une feuille unique de ce feuilleté.

16. Matériau pour pansements sous la forme d'une galette ayant une configuration lobée.

17. Matériau pour pansements suivant la revendication 13, dans lequel la galette comprend deux lobes réunis ensemble par une portion étranglée commune.

18. Pansement suivant l'une quelconque des revendications 2 à 17, dans lequel chacun de ces premier et second bords sont, respectivement, un bord de feuilles séparées d'un feuilleté.

19. Pansement suivant l'une quelconque des revendications 14 à 18, dans lequel les bords sont réunis par thermo-soudure.
